# EUROPEAN PATENT SPECIFICATION

(11) **EP 2 836 579 B1**
(45) Date of publication and mention of the grant of the patent: **25.07.2018**
(21) Application number: 13717656.6
(22) Date of filing: 04.04.2013
(51) Int. Cl.: C11B 9/00, B65F 1/00, C08K 5/00

(54) **MALODOR REDUCTION COMPOSITIONS**
ZUSAMMENSETZUNGEN ZUR GERUCHSREDUZIERUNG
COMPOSITIONS POUR L'ATTÉNUATION DE MAUVAISES ODEURS

(30) Priority: 10.04.2012 US 201261622030 P
(43) Date of publication of application: 18.02.2015
(73) Proprietor: The Procter & Gamble Company, Cincinnati, OH 45202 (US)
(72) Inventor: HOLLINGSHEAD, Judith, Ann, Cincinnati, Ohio 45202 (US); HORENZIAK, Steven, Anthony, Cincinnati, Ohio 45202 (US); MALANYAON, Michael-Vincent, Nario, Cincinnati, Ohio 45202 (US); JEAN-MARY, Fleumingue, Cincinnati, Ohio 45202 (US)
(74) Representative: Siddiquee, Sanaul Kabir
(86) International application number: PCT/US2013/035219
(87) International publication number: WO 2013/154899

(56) References cited:
- EP-A1- 2 110 118
- WO-A1-00/01360
- WO-A1-02/085420
- WO-A1-2006/088878
- WO-A1-2007/096125
- WO-A1-2007/107856
- WO-A1-2009/035740
- WO-A1-2009/131748
- WO-A1-2011/084568
- WO-A2-01/16266
- WO-A2-2012/078626
- GB-A- 2 422 780

## Description

### FIELD OF THE INVENTION

The present invention relates to unscented and low scented malodor reduction compositions, plastic films comprising same and methods of controlling malodors using said compositions.

### BACKGROUND OF THE INVENTION

Unscented or low scented products are desired by consumers as they may be considered more natural and discreet than scented products. Manufacturers of unscented or low scented products for controlling malodors rely on malodor reduction ingredients or other technologies (e.g. filters) to reduce malodors. However, effectively controlling both amine-based malodors (e.g. fish and urine) and sulfur-based malodors (e.g. garlic and onion) may be difficult, and the time required for a product to noticeably reduce malodors may create consumer doubt as to the product's efficacy on malodors. Often times, manufacturers incorporate scented perfumes to help mask these difficult malodors. WO 2009/107856 discloses a malodor reducing composition comprising patchone, suitable for incorporating into plastic film material for trash bags. USPA Serial No. 13/249,616 discloses unscented and low scent malodor reduction compositions that control malodors. Unfortunately, the range of materials used to produce such compositions is more limited than desired. Surprisingly, Applicants recognized that, while perfume raw materials that have high vapor pressures (for example vapor pressures higher than 0.1 torr at 25°C) are expected to produce significant scent as these materials have a higher number of perfume molecules per unit of air, certain high vapor pressure perfume raw materials produce little or no scent and reduce malodor when used at the level taught herein.

### SUMMARY OF THE INVENTION

The present invention relates to unscented and low scented malodor reduction compositions, plastic films comprising same and methods of controlling malodors using said compositions. The malodor reduction compositions are suitable for use in a variety of applications, including use in consumer products, for example, air freshening compositions, laundry detergents, fabric enhancers, surface cleaners, beauty care products, dish care products, diapers, feminine protection articles, and plastic films for garbage bags.

### BRIEF DESCRIPTION OF THE DRAWINGS

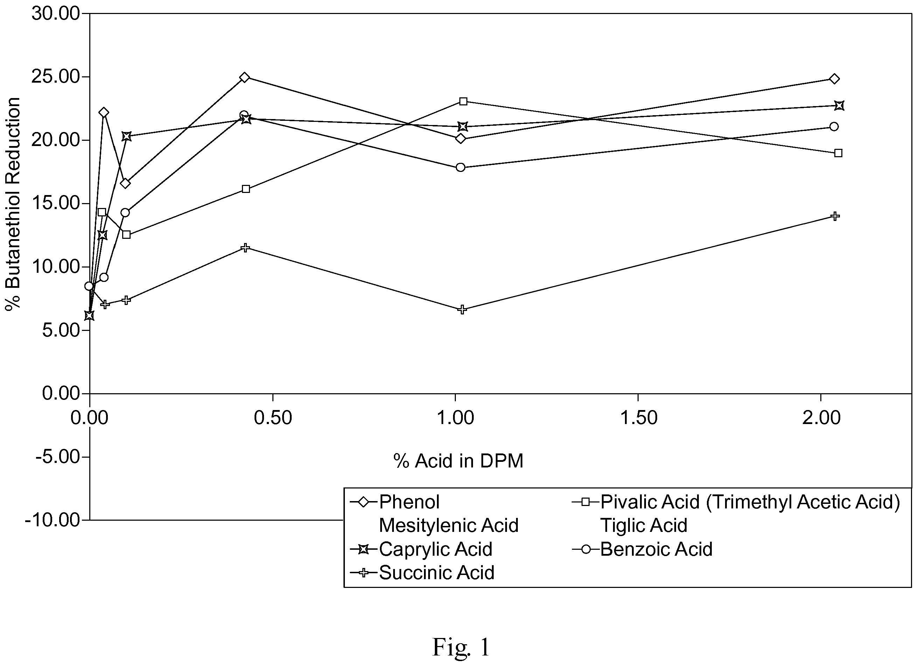

Fig. 1 is a graph showing butanethiol reduction by thiophene carboxaldehyde in combination with various acid catalysts.

### DETAILED DESCRIPTION OF THE INVENTION

As used herein "consumer product" means baby care, beauty care, fabric & home care, family care, feminine care, health care, snack and/or beverage products or devices intended to be used or consumed in the form in which it is sold, and not intended for subsequent commercial manufacture or modification. Such products include but are not limited to diapers, bibs, wipes; products for and/or methods relating to treating hair (human, dog, and/or cat), including, bleaching, coloring, dyeing, conditioning, shampooing, styling; deodorants and antiperspirants; personal cleansing; cosmetics; skin care including application of creams, lotions, and other topically applied products for consumer use; and shaving products, products for and/or methods relating to treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, RX pharmaceuticals, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee.

As used herein "energized system", refers to a system that operates by using an electrical and/or mechanical energy source such as a battery or electrical wall outlet to emit the malodor reduction composition. Examples of such devices include but are limited to liquid electric pluggable type air freshening devices.

As used herein, "malodor" refers to compounds generally offensive or unpleasant to most people, such as the complex odors associated with bowel movements.

As used herein, "neutralize" or "neutralization" refers to the ability of a compound or product to reduce or eliminate malodorous compounds. Odor neutralization may be partial, affecting only some of the malodorous compounds in a given context, or affecting only part of a malodorous compound. A malodorous compound may be neutralized by chemical reaction resulting in a new chemical entity, by sequestration, by chelation, by association, or by any other interaction rendering the malodorous compound less malodorous or non-malodorous. Neutralization is distinguishable from odor masking or odor blocking by a change in the malodorous compound, as opposed to a change in the ability to perceive the malodor without any corresponding change in the condition of the malodorous compound. Malodor neutralization provides a sensory and analytically measurable (e.g. gas chromatograph) malodor reduction. Thus, if a malodor reduction composition delivers genuine malodor neutralization, the composition will reduce malodors in the vapor and/or liquid phase.

As used herein, "non-energized" refers to a system that emits a targeted active passively or without the need for an electrical energy source. Handheld aerosol sprayers and traditional trigger/pump sprayers are considered non-energized systems.

As used herein, "odor blocking" refers to the ability of a compound to dull the human sense of smell.

As used herein, "odor masking" refers to the ability of a compound with a non-offensive or pleasant smell that is dosed such that it limits the ability to sense a malodorous compound. Odor-masking may involve the selection of compounds which coordinate with an anticipated malodor to change the perception of the overall scent provided by the combination of odorous compounds.

As used herein, the terms "a" and "an" mean "at least one".

As used herein, the terms "include", "includes" and "including" are meant to be non-limiting.

Unless otherwise noted, all component or composition levels are in reference to the active portion of that component or composition, and are exclusive of impurities, for example, residual solvents or by-products, which may be present in commercially available sources of such components or compositions.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

### Malodor Reduction Compositions

The present invention relates in part to unscented and low scented malodor reduction compositions. Unscented and low scented malodor reduction compositions of the present invention comprise perfume mixtures that are substantially free of scent. So, rather than simply masking odors, such compositions reduce malodors through odor neutralization and odor blocking technologies. The perceptible perfume scent intensity and malodor efficacy of a composition can be determined using the tests outlined herein.

The malodor reduction compositions of the present invention may include an effective amount of an acid catalyst that can neutralize sulfur-based malodors. It has been found that certain mild acids have an impact on aldehyde reactivity with thiols in the liquid and vapor phase. It has been found that the reaction between thiol and aldehyde is a catalytic reaction that follows the mechanism of hemi-thioacetal and thioacetal formation path. When the present malodor reduction composition contains an acid catalyst and contacts a sulfur-based malodor, volatile aldehydes react with thiol. This reaction may form a thiol acetal compound, thus, neutralizing the sulfur-based odor. Without an acid catalyst, only hemi-thiolacetal is formed.

A malodor reduction composition is provided comprising a perfume mixture comprising an effective amount of methyl palmitate, farnesol, vetivert acetate, undecylenic aldehyde, terpinyl acetate, methyl iso-eugenol, phenyl acetaldehyde dimethyl acetal, patchone and optionally a material selected from the group consisting of benzophenones, diphenyl oxide, melozone, iso nonyl acetate, cedryl methyl ether and mixtures thereof, said malodor reduction composition or said perfume mixture being optionally encapsulated is disclosed. Said malodor reduction composition is identified herein as "Malodor Reduction Composition 1".

In one aspect, said malodor reduction composition's perfume mixture is present in an amount up to 100%, by weight of the malodor reduction composition, alternatively from 5% to 100%, alternatively from about 10% to about 100%, alternatively from about 30% to about 100%, alternatively from about 50% to about 100%, alternatively from about 70% to about 100%, alternatively from about 80% to about 100%, alternatively from about 0.001% to about 5%, alternatively from about 0.001% to about 2%, alternatively from about 0.001% to about 0.5%, alternatively from about 0.001% to about 0.3%, alternatively from about 0.001% to about 0.1%, alternatively about 0.001%, by weight of the malodor reduction composition.

In one aspect, said malodor reduction composition comprises cedryl methyl ether, florhydral, helional, vertofix coeur, and mixtures thereof.

In one aspect, said malodor reduction composition's perfume mixture comprises at least one aldehyde selected from the group consisting of floral super, 2-ethoxy Benzylaldehyde, 2-isopropyl-5-methyl-2-hexenal, 5-methyl Furfural, 5-methyl-thiophene-carboxaldehyde, p-anisaldehyde, benzylaldehyde, cinnamic aldehyde, decyl aldehyde, Ligustral, Lyral, Melonal, o-anisaldehyde, P.T. Bucinal, thiophene carboxaldehyde, trans-4-decenal, trans trans 2,4-nonadienal, undecyl aldehyde, and mixtures thereof

In one aspect, said malodor reduction composition's perfume mixture comprises from 5% to 100%, from about 8% to about 70%, from about 10% to about 50% or even from about 12% to about 30% benzophenone, methyl palmitate, farnesol, vetivert acetate, and undecylenic aldehyde.

In one aspect, said malodor reduction composition's perfume mixture comprises a aldehyde mixture selected from the group consisting of Accord A, Accord B, Accord C, and mixtures thereof. Such accords are given below:

**Accord A**

| **Material** | **Wt. % (of the aldehydes in the perfume mixture)** | **CAS Number** | **VP(torr) @25°C** |
|---|---|---|---|
| Intreleven Aldehyde | 5.000 | 112-45-8 | 0.060 |
| Florhydral | 10.000 | 125109-85-5 | 0.008 |
| Floral Super | 25.000 | 71077-31-1 | 0.030 |
| Scentenal | 10.000 | 86803-90-9 | 0.010 |
| Cymal | 25.000 | 103-95-7 | 0.007 |
| o-anisaldehyde | 25.000 | 135-02-4 | 0.032 |

**Accord B**

| **Material** | **Wt. % (of the aldehydes in the perfume mixture)** | **CAS Number** | **VP (torr) @25°C** |
|---|---|---|---|
| Intreleven Aldehyde | 2.000 | 112-45-8 | 0.060 |
| Florhydral | 20.000 | 125109-85-5 | 0.008 |
| Floral Super | 10.000 | 71077-31-1 | 0.030 |
| Scentenal | 5.000 | 86803-90-9 | 0.010 |
| Cymal | 25.000 | 103-95-7 | 0.007 |
| Floralozone | 10.000 | 67634-14-4 | 0.005 |
| Adoxal | 1.000 | 141-13-9 | 0.007 |
| Methyl Nonyl Acetaldehyde | 1.000 | 110-41-8 | 0.030 |
| Melonal | 1.000 | 106-72-9 | 0.670 |
| o-anis aldehyde | 25.000 | 135-02-4 | 0.032 |

**Accord C**

| **Material** | **Wt. % (of the aldehydes in the perfume mixture)** | **CAS Number** | **VP (torr) @25°C** |
|---|---|---|---|
| Intreleven Aldehyde | 2.000 | 112-45-8 | 0.060 |
| Florhydral | 10.000 | 125109-85-5 | 0.008 |
| Floral Super | 5.000 | 71077-31-1 | 0.030 |
| Scentenal | 2.000 | 86803-90-9 | 0.010 |
| Cymal | 15.000 | 103-95-7 | 0.007 |
| Floralozone | 12.000 | 67634-14-4 | 0.005 |
| Adoxal | 1.000 | 141-13-9 | 0.007 |
| Methyl Nonyl Acetaldehyde | 1.000 | 110-41-8 | 0.030 |
| Melonal | 1.000 | 106-72-9 | 0.670 |
| Flor Acetate | 11.800 | 5413-60-5 | 0.060 |
| Frutene | 7.000 | 17511-60-3 | 0.020 |
| Helional | 5.000 | 1205-17-0 | 0.0005 |
| Bourgeonal | 2.000 | 18127-01-0 | 0.004 |
| Linalool | 10.000 | 78-70-6 | 0.050 |
| Benzaldehyde | 0.200 | 100-52-7 | 1.110 |
| o-anis aldehyde | 15.000 | 135-02-4 | 0.320 |

Accords A, B, or C can be formulated in with the malodor reduction composition's perfume mixture, for example, the perfume mixtures outlined in Tables 1 to 7 of the present specification in an amount of about 5% to about 50%, alternatively about 5% to about 40%, alternatively about 5% to about 30%, alternatively about 5% to about 20%, alternatively about 5% to about 10%, by weight of the perfume mixture.

In one aspect, said malodor reduction composition's perfume mixture comprises 1% to 10% of Accord A, by weight of said perfume mixture.

In one aspect, said malodor reduction composition comprises, based on total malodor reduction composition weight, from about 0.05% to about 5%, alternatively 0.1% to 1.5% alternatively about 0.1% to about 1.0%, alternatively about 0.1% to about 0.5%, alternatively about 0.1% to about 0.4%, alternatively about 0.4% to about 1.5%, alternatively about 0.4% of an acid catalyst.

In one aspect, said malodor reduction composition acid catalyst may be a weak acid. A weak acid is characterized by an acid dissociation constant, Kₐ, which is an equilibrium constant for the dissociation of a weak acid; the pKa being equal to minus the decimal logarithm of Kₐ.

In one aspect, said malodor reduction composition's acid catalyst has a vapor pressure of from 0.001 torr to about 38 torr, alternatively 0.001 to 20 torr, alternatively about 0.001 torr to about 14 torr, alternatively about 0.01 to about 2 torr, alternatively from about 0.001 torr to about 1 torr, alternatively from about 0.001 torr to about 0.020 torr, alternatively about 0.005 torr to about 0.020 torr, alternatively about 0.010 torr to about 0.020 torr, measured at 25°C.

In one aspect, said malodor reduction composition's acid catalyst is a carboxylic acid.

In one aspect, said malodor reduction composition's acid catalyst is 5-methyl thiophene carboxylic acid.

In one aspect, said malodor reduction composition's acid catalyst has a pKa of 1 to 7, from about 3 to about 6, from about 4 to about 6.5, from about 4.0 to about 6.0, from about 4.3 and 5.7, from about 4.5 to about 5, or from about 4.7 to about 4.9.

In one aspect, said malodor reduction composition's acid catalyst may be selected from Formic Acid, Acetic Acid, Trimethyl Acetic Acid, Phenol (alkaline in liquid apps yet acidic in vapor phase), Tiglic acid, Caprylic acid, 5-Methyl thiophene carboxylic acid, Succinic acid, Benzoic acid, and/or Mesitylenic acid.

In one aspect, it may be desirable to select an acid catalyst that provides a neutral scent. Such acid catalysts may have a vapor pressure of about 0.001 torr to about 0.020 torr, measured at 25°C, alternatively about 0.005 torr to about 0.020 torr, alternatively about 0.010 torr to about 0.020 torr. Non-limiting examples of such acid catalyst include succinic acid and benzoic acid.

In an acetic acid system, the present malodor reduction composition may include about 0.4% of acetic acid (50:50 thiophene carboxaldehye (TC): dipropylene glycol methyl ether (DPM), 0.4% acetic acid). Data is provided in the table below.

| **Sample Formulated** | **Actual % acetic acid in DPM** | **% Butanethiol reduction @ 30 min.** |
|---|---|---|
| 50:50 TC:DPM 0% Acetic Acid | 0.00 | 12.00 |
| 50:50 TC:DPM 0.05% Acetic Acid | 0.04 | 14.65 |
| 50:50 TC:DPM 0.1% Acetic Acid | 0.10 | 25.66 |
| 50:50 TC:DPM 0.2% Acetic Acid | 0.42 | 34.68 |
| 50:50 TC:DPM 0.5% Acetic Acid | 1.00 | 24.79 |
| 50:50 TC:DPM 1.0% Acetic Acid | 2.00 | 7.26 |

When an acid catalyst is present with an aldehyde, the acid catalyst may increase the efficacy of the aldehyde on malodors in comparison to the malodor efficacy of the aldehyde on its own. For example, 1% aldehyde and 1.5% benzoic acid provides malodor removal benefit equal to or better than 5% aldehyde alone.

When formulated in a aqueous solution, the malodor reduction composition may have a pH from about 3 to about 8, alternatively from about 4 to about 7, alternatively from about 4 to about 6.

In one aspect, said malodor reduction composition comprises an ingredient selected from the group consisting of: odor masking agents, odor blocking agents, diluents, and mixtures thereof.

A malodor reduction composition comprising a perfume mixture comprising styrax coeur and an effective amount of at least one perfume material selected from the group consisting of: terpinyl acetate, methyl iso-eugenol, phenyl acetaldehyde dimethyl acetal, patchone, and mixtures thereof said malodor reduction composition or said perfume mixture being optionally encapsulated is disclosed, but does not form part of the present invention. Said malodor reduction composition is identified as "Malodor Reduction Composition 2".

A malodor reduction composition comprising a perfume mixture comprising from about 5% to about 100%, from about 8% to about 70%, from about 10% to about 50% or even from about 12% to about 30% by weight of said perfume mixture, of at least two, at least three or at least four perfume materials selected from the group consisting of terpinyl acetate, methyl Iso-eugenol, phenyl acetaldehyde dimethyl acetal, and patchone, said malodor reduction composition or said perfume mixture being optionally encapsulated is disclosed, but does not form part of the present invention. Said malodor reduction composition is identified as "Malodor Reduction Composition 3".

In one aspect, said malodor reduction composition's perfume mixture comprises cedryl methyl ether, florhydral, helional, undecylenic aldehyde, vetivert acetate, vertofix couer, and mixtures thereof.

### Additional Perfume Materials

Additional perfume materials that may be used include Benzophenone, Methyl Palmitate, Farnesol, Vetivert Acetate, Cedryl Methyl Ether, Vertofix Couer (methyl cedrylone), and mixtures thereof. Suitable perfume materials may also include Helional (alpha-methyl-3,4-(methylenedioxy)-hydrocinnamaldehyde), Florhydral, Undecylenic Aldehyde, Adoxal (2,6,10-Trimethyl-9-undecenal), Bourgeonal (4-t-butylbenzenepropionaldehyde), Cymal, Florhydral (3-(3-isopropyl-phenyl)-butyraldehyde), Citronellal (3,7-dimethyl 6-octenal), Floralozone (para-ethyl-alpha,alpha-dimethyl hydrocinnamaldehyde), Floral Super, Pino Acetaldehyde, Styrax Coeur.

Suitable perfume materials may also include volatile aldehydes or reactive aldehydes (RA) including, but not limited to, Lilestralis 33 (2-methyl-4-t-butylphenyl)propanal), Cinnamic aldehyde, cinnamaldehyde (phenyl propenal, 3-phenyl-2-propenal), Citral, Geranial, Neral (dimethyloctadienal, 3,7-dimethyl-2,6-octadien-1-al), Cyclal C (2,4-dimethyl-3-cyclohexen-1-carbaldehyde), cyclamen aldehyde, Cyclosal, Lime aldehyde (Alpha-methyl-p-isopropyl phenyl propyl aldehyde), Methyl Nonyl Acetaldehyde, aldehyde C12 MNA (2-methyl-1-undecanal), Hydroxycitronellal, citronellal hydrate (7-hydroxy-3,7-dimethyl octan-1-al), hydrocinnamaldehyde (3-phenylpropanal, 3-phenylpropionaldehyde), Intreleven aldehyde (undec-10-en-1-al), Ligustral, Trivertal (2,4-dimethyl-3-cyclohexene-1-carboxaldehyde), Jasmorange, satinaldehyde (2-methyl-3-tolylproionaldehyde, 4-dimethylbenzenepropanal), Lyral (4-(4-hydroxy-4-methyl pentyl)-3-cyclohexene-1-carboxaldehyde), Melonal (2,6-Dimethyl-5-Heptenal), Methoxy Melonal (6-methoxy-2,6-dimethylheptanal), methoxycinnamaldehyde (trans-4-methoxycinnamaldehyde), Myrac aldehyde isohexenyl cyclohexenyl-carboxaldehyde, trifernal ((3-methyl-4-phenyl propanal, 3-phenyl butanal), lilial, P.T. Bucinal, lysmeral, benzenepropanal (4-tert-butyl-alpha-methyl-hydrocinnamaldehyde), Dupical, tricyclodecylidenebutanal (4-Tricyclo5210-2,6decylidene-8butanal), Melafleur (1,2,3,4,5,6,7,8-octahydro-8,8-dimethyl-2-naphthaldehyde), Methyl Octyl Acetaldehyde, aldehyde C-11 MOA (2-methyl deca-1-al), Onicidal (2,6,10-trimethyl-5,9-undecadien-1-al), Muguet aldehyde 50 (3,7-dimethyl-6-octenyl) oxyacetaldehyde), phenylacetaldehyde, Mefranal (3-methyl-5-phenyl pentanal), Triplal, Vertocitral dimethyl tetrahydrobenzene aldehyde (2,4-dimethyl-3-cyclohexene-1-carboxaldehyde), 2-phenylproprionaldehyde, Hydrotropaldehyde, Canthoxal, anisylpropanal 4-methoxy-alpha-methyl benzenepropanal (2-anisylidene propanal), Cylcemone A (1,2,3,4,5,6,7,8-octahydro-8,8-dimethyl-2-naphthaldehyde), and Precylcemone B (1-cyclohexene-1-carboxaldehyde).

Suitable aldehydes may also include acetaldehyde (ethanal), pentanal, valeraldehyde, amylaldehyde, Scentenal (octahydro-5-methoxy-4,7-Methano-1H-indene-2-carboxaldehyde), propionaldehyde (propanal), Cyclocitral, beta-cyclocitral, (2,6,6-trimethyl-1-cyclohexene-1-acetaldehyde), Iso Cyclocitral (2,4,6-trimethyl-3-cyclohexene-1-carboxaldehyde), isobutyraldehyde, butyraldehyde, isovaleraldehyde (3-methyl butyraldehyde), methylbutyraldehyde (2-methyl butyraldehyde, 2-methyl butanal), Dihydrocitronellal (3,7-dimethyl octan-1-al), 2-Ethylbutyraldehyde, 3-Methyl-2-butenal, 2-Methylpentanal, 2-Methyl Valeraldehyde, Hexenal (2-hexenal, trans-2-hexenal), Heptanal, Octanal, Nonanal, Decanal, Tridecanal, 2-Dodecanal, Methylthiobutanal, Glutaraldehyde, Pentanedial, Glutaric aldehyde, Heptenal, cis or trans-Heptenal, Undecenal (2-, 10-), 2,4-octadienal, Nonenal (2-, 6-), Decenal (2-, 4-), 2,4-hexadienal, 2,4-Decadienal, 2,6-Nonadienal, Octenal, 2,6-dimethyl 5-heptenal, 2-isopropyl-5-methyl-2-hexenal, Trifernal, beta methyl Benzenepropanal, 2,6,6-Trimethyl-1-cyclohexene-1-acetaldehyde, phenyl Butenal (2-phenyl 2-butenal), 2-Methyl-3-(p-isopropylphenyl)-propionaldehyde, 3-(p-isopropylphenyl)-propionaldehyde, p-Tolylacetaldehyde (4-methylphenylacetaldehyde), Anisaldehyde (p-methoxybenzene aldehyde), Benzaldehyde, Vernaldehyde (1-Methyl-4-(4-methylpentyl)-3-cyclohexenecarbaldehyde), Heliotropin (piperonal) 3,4-Methylene dioxy benzaldehyde, alpha-Amylcinnamic aldehyde, 2-pentyl-3-phenylpropenoic aldehyde, Vanillin (4-methoxy 3-hydroxy benzaldehyde), Ethyl vanillin (3-ethoxy 4-hydroxybenzaldehyde), Hexyl Cinnamic aldehyde, Jasmonal H (alpha-n-hexylcinnamaldehyde), Acalea (p-methyl-alpha-pentylcinnamaldehyde), methylcinnamaldehyde, alpha-Methylcinnamaldehyde (2-methyl 3-pheny propenal), alpha-hexylcinnamaldehyde (2-hexyl 3-phenyl propenal), Salicylaldehyde (2-hydroxy benzaldehyde), 4-ethyl benzaldehyde, Cuminaldehyde (4-isopropyl benzaldehyde), Ethoxybenzaldehyde, 2,4-dimethylbenzaldehyde, Veratraldehyde (3,4-dimethoxybenzaldehyde), Syringaldehyde (3,5-dimethoxy 4-hydroxybenzaldehyde), Catechaldehyde (3,4-dihydroxybenzaldehyde), Safranal (2,6,6-trimethyl-1,3-diene methanal), Myrtenal (pin-2-ene-1-carbaldehyde), Perillaldehyde L-4(1-methylethenyl)-1-cyclohexene-1-carboxaldehyde), 2,4-Dimethyl-3-cyclohexene carboxaldehyde, 2-Methyl-2-pentenal, 2-methylpentenal, pyruvaldehyde, formyl Tricyclodecan, Mandarin aldehyde, Cyclemax, Corps Iris, Maceal, and Corps 4322.

In some embodiments, the malodor reduction composition includes fast reacting aldehydes. "Fast reacting aldehydes" refers to aldehydes that either (1) reduce amine odors by 20% or more in less than 40 seconds; or (2) reduce thiol odors by 20% or more in less than 30 minutes. Fast reacting aldehydes can be identified by the method outlined in the Example outlined herein, titled "Analytical Test - Effect of aldehydes on amine-based and sulfur-based malodors".

Table 1 shows an embodiment of a perfume mixture which is not according to the present invention.

**Mixture Composition A**

| **Material name** | **Weight %** | **CAS #** |
|---|---|---|
| Diphenyl oxide | 0.5 | 101-84-8 |
| Melozone | 0.5 | 30772-79-3 |
| Undecylenic Aldehyde | 1 | 112-45-8 |
| Benzophenone | 5 | 119-61-9 |
| Iso nonyl acetate | 5 | 58430-94-7 |
| Undecyl aldehyde | 5 | 112-45-8 |
| Cedryl Methyl ether | 10 | 19870-74-7 |
| Methyl iso eugenol | 23 | 93-16-3 |
| Phenyl acetaldehyde dimethyl acetal | 25 | 101-48-4 |
| Terpinyl acetate | 25 | 80-26-2 |
| TOTAL | 100.00 | |

Table 2 shows an embodiment of a perfume mixture suitable for the malodor reduction compositions of the present invention.

Table 3 shows an embodiment of a perfume mixture which is not according to the present invention.

**Table 2 - Low Scented Mixture Composition B**

| **Material name** | **Weight %** | **CAS #** |
|---|---|---|
| 5-Cyclohexadecen-1-One | 4 | 37609-25-9 |
| Cedryl Methyl Ether | 0.5 | 19870-74-7 |
| Florhydral | 1 | 125109-85-5 |
| Helional | 0 | 1205-17-0 |
| Vertofix Coeur | 20 | 32388-55-9 |
| Undecylenic Aldehyde | 0.2 | 112-45-8 |
| Methyl palmitate | 15 | 112-39-0 |
| Vetivert Acetate | 0.5 | 68917-34-0 |
| Farnesol | 15 | 4602-84-0 |
| Adoxal | 0.8 | 141-13-9 |
| Methyl Iso Eugenol | 10 | 93-16-3 |
| Terpinyl Acetate | 10 | 80-26-2 |
| Phenyl Acetaldehyde Dimethyl Acetal | 10 | 101-48-4 |
| Patchon | 13 | 98-52-2 |
| TOTAL | 100.00 | |

**Table 3 - Low Scented Mixture Composition C**

| **Material Name** | **Weight %** | **CAS #** |
|---|---|---|
| Cedryl Methyl Ether | 0.50 | 19870-74-7 |
| Florhydral | 1.00 | 125109-85-5 |
| Helional | 0.00 | 1205-17-0 |
| Vertofix Coeur | 20.00 | 32388-55-9 |
| Undecylenic Aldehyde | 0.20 | 112-45-8 |
| Methyl palmitate | 15.00 | 112-39-0 |
| Vetivert Acetate | 1.00 | 68917-34-0 |
| Farnesol | 15.00 | 4602-84-0 |
| Adoxal | 0.80 | 141-13-9 |
| Methyl Iso Eugenol | 16.50 | 93-16-3 |
| Terpinyl Acetate | 20.00 | 80-26-2 |
| Phenyl Acetaldehyde Dimethyl Acetal | 10.00 | 101-48-4 |
| TOTAL | 100.00 | |

Table 4 shows yet another embodiment of a perfume mixture suitable for the malodor reduction composition of the present invention.

**Table 4 - Plastic Low Scented Mixture Compositions D and E**

| **Material Name** | **Composition D Weight %** | **Composition E Weight %** | **CAS #** |
|---|---|---|---|
| 5-Cyclohexadecen-1-One | 4.00 | 4.00 | 37609-25-9 |
| Cedryl Methyl Ether | 0.50 | 0.50 | 19870-74-7 |
| Florhydral | 1.00 | 1.00 | 125109-85-5 |
| Helional | 0.00 | 0.00 | 1205-17-0 |
| Vertofix Coeur | 14.50 | 20.00 | 32388-55-9 |
| Undecylenic Aldehyde | 0.20 | 0.20 | 112-45-8 |
| Methyl palmitate | 16.00 | 15.00 | 112-39-0 |
| Vetivert Acetate | 0.50 | 0.50 | 68917-34-0 |
| Farnesol | 15.00 | 15.00 | 4602-84-0 |
| Adoxal | 0.80 | 0.80 | 141-13-9 |
| Methyl Iso Eugenol | 10.00 | 10.00 | 93-16-3 |
| Terpinyl Acetate | 10.00 | 10.00 | 80-26-2 |
| Phenyl Acetaldehyde Dimethyl Acetal | 10.00 | 10.00 | 101-48-4 |
| Patchon | 13.00 | 13.00 | 98-52-2 |
| Styrax Coeur | 4.5 | 0 | 8046-19-3 |
| TOTAL | 100.00 | 100.00 | |

Table 5 shows yet another embodiment of a perfume mixture suitable for an aerosol-based malodor reduction composition, which is not according to the present invention.

**Table 5 - Low Scented Mixture Composition F**

| **Material Name** | **Weight %** | **CAS #** |
|---|---|---|
| Diphenyl Oxide | 0.14 | 101-84-8 |
| Melozone | 0.14 | |
| Undecylenic Aldehyde | 0.27 | 112-45-8 |
| Iso Nonyl Acetate | 1.35 | 58430-94-7 |
| Undecyl Aldehyde | 1.35 | 112-45-8 |
| Cedryl Methyl Ether | 2.70 | 19870-74-7 |
| Methyl Iso Eugenol | 6.21 | 93-16-3 |
| Phenyl Acetaldehyde Dimethyl Acetal | 6.75 | 101-48-4 |
| Terpinyl Acetate | 7.56 | 80-26-2 |
| Vertofix Coeur | 0.54 | 32388-55-9 |
| Other Perfume Materials | Balance to 100% | |

Table 6 shows yet another embodiment of a perfume mixture suitable for a non-energized, membrane type air freshener (e.g. Febreze® Set & Refresh™ air freshener), which is not according to the present invention.

**Table 6 - Low Scented Mixture Composition G**

| **Material Name** | **Weight %** | **CAS #** |
|---|---|---|
| Diphenyl Oxide | 0.09 | 101-84-8 |
| Melozone | 0.09 | |
| Undecylenic Aldehyde | 0.18 | 112-45-8 |
| Iso Nonyl Acetate | 0.90 | 58430-94-7 |
| Undecyl Aldehyde | 0.90 | 112-45-8 |
| Cedryl Methyl Ether | 1.80 | 19870-74-7 |
| Methyl Iso Eugenol | 4.14 | 93-16-3 |
| Phenyl Acetaldehyde Dimethyl Acetal | 4.50 | 101-48-4 |
| Terpinyl Acetate | 5.04 | 80-26-2 |
| Vertofix Coeur | 0.36 | 32388-55-9 |
| Other Perfume Materials | Balance to 100% | |

Table 7 shows yet another embodiment of a perfume mixture suitable for a liquid electric, pluggable type air freshener (e.g. Febreze® Noticeables™ air freshener), which is not according to the present invention.

**Table 7 - Low Scented Mixture Composition H**

| **Material Name** | **Weight %** | **CAS #** |
|---|---|---|
| Diphenyl Oxide | 0.05 | 101-84-8 |
| Melozone | 0.05 | |
| Undecylenic Aldehyde | 0.10 | 112-45-8 |
| Iso Nonyl Acetate | 0.50 | 58430-94-7 |
| Undecyl Aldehyde | 0.50 | 112-45-8 |
| Cedryl Methyl Ether | 1.00 | 19870-74-7 |
| Methyl Iso Eugenol | 2.30 | 93-16-3 |
| Phenyl Acetaldehyde Dimethyl Acetal | 2.50 | 101-48-4 |
| Terpinyl Acetate | 2.80 | 80-26-2 |
| Vertofix Coeur | 0.20 | 32388-55-9 |
| Other Perfume Materials | Balance to 100% | |

In some embodiments, the malodor reduction composition includes a mixture of perfume materials identified in Tables 1-7 along with a mixture of two or more aldehydes selected from the group consisting of 2-ethoxy Benzylaldehyde, 2-isopropyl-5-methyl-2-hexenal, 5-methyl Furfural, 5-methyl-thiophene-carboxaldehyde, p-anisaldehyde, Benzylaldehyde, Cinnamic aldehyde, Decyl aldehyde, Ligustral, Lyral, Melonal, o-anisaldehyde, P.T. Bucinal, Thiophene carboxaldehyde (TC), trans-4-Decenal, trans trans 2,4-Nonadienal, Undecyl aldehyde, and mixtures thereof.

In some embodiments where volatility is not important for neutralizing a malodor, the present invention may include poly-aldehydes, for example, di-, tri-, tetra-aldehydes. Such embodiments may include laundry detergents, additive, and the like for leave-on, through the wash, and rinse-off type of applications.

### Process of Making Encapsulates

Methods of making suitable encapsulated malodor reduction compositions, for example condensation processes, as well as suitable shell materials for such encapsulated malodor reduction compositions are described in US Patent No. 6,869,923 B1 and US Published Patent Applications Nos. 2005/0276831 A1 and 2007/020263 A1. Such shell materials include acrylates, acrylics, aminoplast materials such as melamine formaldehyde material and combinations thereof. Suitable equipment for use in the processes disclosed herein may include continuous stirred tank reactors, homogenizers, turbine agitators, recirculating pumps, paddle mixers, ploughshear mixers, ribbon blenders, vertical axis granulators and drum mixers, both in batch and, where available, in continuous process configurations, spray dryers, and extruders. Such equipment can be obtained from Lodige GmbH (Paderborn, Germany), Littleford Day, Inc. (Florence, Kentucky, U.S.A.), Forberg AS (Larvik, Norway), Glatt Ingenieurtechnik GmbH (Weimar, Germany), Niro (Soeborg, Denmark), Hosokawa Bepex Corp. (Minneapolis, Minnesota, U.S.A.), Arde Barinco (New Jersey, U.S.A.).

### Optional Ingredients

The malodor reduction composition may, optionally, include odor masking agents and/or diluents.

Water and surfactants may also be present in any amount for the composition to make an aqueous solution. In some embodiments, water may be present in an amount of about 85% to 99.5%, alternatively about 90% to about 99.5%, alternatively about 92% to about 99.5%, alternatively about 95%, by weight of said malodor reduction composition. Water containing a small amount of low molecular weight monohydric alcohols, e.g., ethanol, methanol, and isopropanol, or polyols, such as ethylene glycol and propylene glycol, can also be useful.

The malodor reduction composition may also comprise 100% of an unscented or low scented perfume mixture according to the present invention.

The malodor reduction composition of the present invention may be combined with one or more diluents. When combined with said diluents, the malodor reduction composition may comprise from about 1 to about 80% or from about 5% to about 50% or from about 10% to about 30% of the total mixture. For use in the present invention, diluents with low scent intensity are preferred, but not required. Exemplary diluents include DBE-LVP (Mixed aliphatic ester fluid (CAS#1119-40-0 and CAS# 627-93-0 from INVISTA), dipropylene glycol methyl ether, 3-methoxy-3-methyl-1-butanol, isononyl acetate, benzyl alcohol, florol, dioctyl adipate (CAS#123-79-5), Tripropylene glycol Methyl ether (CAS # 25498-49-1), Dow Corning 200(R) Fluid, 1.5 CST®. (from the Dow Corning Co.), Dipropylene glycol n-propyl ether, Xiameter(R) PMX-200 Silicone Fluid 1.5CS® (from the Dow Corning Co.), cellulose, Ethyl ether and mixtures thereof.

### Articles of Manufacture Comprising Malodor Reduction Compositions and Methods of Using Such Compositions

The malodor reduction composition of the present invention may be used in a wide variety of applications that neutralize malodors in the vapor and/or liquid phase.

A plastic film comprising Malodor Reduction Composition 11 forms one aspect of the present invention.

In one aspect, said plastic film comprises low density polyethylene (LDPE), linear LDPE (LLDPE), high density polyethylene, and/or compostable films.

In one aspect, said plastic film comprises from 0.5 mg to 100 mg, from about 0.5 mg to about 50 mg, from 5 mg to 30 mg or even 5 mg to 15 mg of said malodor reduction composition per 20 grams of said plastic film.

A method of controlling malodors comprising contacting the material comprising a malodor with Malodor Reduction Composition 1. In one aspect, said method's contacting step comprises contacting said material containing a malodor with 1 mg to 50 mg, from 3 mg to 30 mg, or from 5 mg to 20 mg of said composition per 20 grams of said material containing a malodor.

In some embodiments, the malodor reduction composition may also be used with an energized system.

In other embodiments, the malodor reduction composition may be formulated for use in non-energized vapor-phase systems. For non-energized systems, the vapor pressure of the volatile aldehydes may be about 0.001 torr to about 20 torr alternatively, about 0.01 torr to about 20 torr, alternatively about 0.05 torr to about 10 torr, measured at 25°C. Non-limiting examples of a non-energized vapor phase system are passive air freshening diffusers such as those known by the trade name Renuzit® Crystal Elements; and aerosol sprays such as fabric and air freshening sprays and body deodorants.

In other embodiments, the malodor reduction composition may be formulated for use in a liquid phase system. For such systems, the vapor pressure may be about 0 torr to about 20 torr, alternatively about 0.0001 torr to about 10 torr, measured at 25°C. Non-limiting examples of a liquid phase system are liquid laundry products, such as laundry detergents and additives; dish detergents; personal hygiene products such as body washes, shampoos, conditioners.

The malodor reduction composition may also be loaded onto or into known substrates according to known methods. Suitable substrates may include wovens and non-wovens (e.g. cellulose fibers for paper products, sponges, and the like). Such substrates may be used to manufacture diapers; baby wipes; adult incontinence products; feminine hygiene products such as sanitary napkins and tampons; cleaning wands for toilets; pet food packaging; paper towels; facial tissues; and the like.

Suitable substrates may also include commercially available films including (LDPE), (LLDPE), (HDPE), plastomers, elastomers, ethylene vinyl acetate, ethyl methacrylates, polymethylpentene copolymers, polyisobutylenes, polyolefin isomers, cyclic olefin copolymers, polyethylene, polypropylene, poly lactic acid based films, polyhydroxy alcohol based films, polyhydroxy butyrate/valerate, polyesters, thermoplastic starch, and combinations thereof. These plastic films may be used to manufacture non-disposable composting containers, trash bags, storage bags, and the like. The malodor reduction composition may also be used in connection with commercial or industrial septic tanks or sewage treatment equipment.

### EXAMPLES

### Example 1 (freshening composition for non-energized article of manufacture)

| | Example 1-A | Example 1-B | Example 1-C |
|---|---|---|---|
| Perfume* | 5-50 | 8-30 | 10-20 |
| Malodor Reduction Composition A of Table 1 | 5-50 | 10-30 | 10-20 |
| Diluent A as given below | Balance | Balance | Balance |

| | | | |
|---|---|---|---|
| *Any desired perfume can be used. | | | |

Ranges are weight % based on total composition

**Diluent A:**

| | % range |
|---|---|
| Benzyl Alcohol | 40-70 |
| Iso Nonyl Acetate | 10-50 |
| Pyranol | 1-30 |

A Febreze® Set & Refresh™ non-energized air freshener is filled with 5.5ml of the composition above. The resulting air freshener has low scent intensity and is effective at reducing malodor.

### Example 2 (freshening composition for energized article of manufacture)

| | Example 2-A | Example 2-B | Example 2-C |
|---|---|---|---|
| Perfume* | 5-50 | 8-30 | 10-20 |
| Malodor Reduction Composition A of Table 1 | 5-50 | 10-30 | 10-20 |
| Diluent B as given below | Balance | Balance | Balance |

| | | | |
|---|---|---|---|
| *Any desired perfume can be used | | | |

**Diluent B:**

| | % range |
|---|---|
| Benzyl Alcohol | 10-50 |
| Iso Nonyl Acetate | 1-30 |
| Dipropylene glycol methyl ether | 40-70 |

A Febreze® Noticeables™ energized air freshening device is filled with the composition above. The resulting air freshener has low scent intensity and is effective at reducing malodor.

### Analytical Test - Effect of volatile aldehydes on amine-based and sulfur-based malodors

Malodor standards are prepared by pipeting 1 mL of n-butylamine (amine-based malodor) or 1-butanethiol (sulfur-based malodor) into a 1.2 liter gas sampling bag. The bag is then filled to volume with nitrogen and allowed to sit for at least 12 hours to equilibrate.

A 1 µL sample of each volatile aldehyde listed in Table 7 and of each Accord (A, B, and C) is pipeted into individual 10 mL silanized headspace vials. The vials are sealed and allowed to equilibrate for at least 12 hours. Repeat 4 times for each sample (2 for butylamine analysis and 2 for butanethiol analysis).

After the equilibration period, 1.5 mL of the target malodor standard is injected into each 10 mL vial. For thiol analysis, the vials containing a sample +malodor standard are held at room temperature for 30 minutes. Then, a 1 mL headspace syringe is then used to inject 250 µL of each sample/thiol malodor into a GC/MS split/splitless inlet. For amine analysis, a 1 mL headspace syringe is used to inject 500 µL of each sample/amine malodor immediately into the GC/MS split/splitless inlet. A GC pillow is used for the amine analysis to shorten the run times.

Samples are then analyzed using a GC/MS with a DB-5, 20 m, 1 µm film thickness column with an MPS-2 autosampler equipment with static headspace function. Data is analyzed by ion extraction on each total ion current (56 for thiol and 30 for amine) and the area is used to calculate the percent reduction from the malodor standard for each sample.

Table 8 shows the effect of certain aldehydes on neutralizing amine-based and sulfur based malodors at 40 seconds and 30 minutes, respectively.

**Table 8**

| **Perfume Raw Material (R-CHO)** | **At least 20% butylamine reduction at 40 secs.?** | **At least 20% butanethiol reduction at 30 mins.?** |
|---|---|---|
| 2-ethoxy benzylaldehyde | Yes | Yes |
| 2-isopropyl-5-methyl-2-hexenal | Yes | Yes |
| Adoxal | Yes | No |
| Cinnamic aldehyde | Yes | Yes |
| Floral Super | Yes | Yes |
| Florhydral | Yes | Yes |
| o-anisaldehyde | Yes | Yes |
| Pino acetaldehyde | Yes | Yes |
| Trans-4-decenal | Yes | Yes |

Table 9 shows the percent reduction of butylamine and butaniethiol at 40 seconds and 30 minutes, respectively, for Accords A, B, and C.

**Table 9**

| **Accord** | **% reduction of butylamine at 40 secs.** | **% reduction of butanethiol at 30 mins.** |
|---|---|---|
| Accord A | 76.58 | 25.22 |
| Accord B | 51.54 | 35.38 |
| Accord C | 65.34 | 24.98 |

### Analytical Test - Effect of acid catalysts on sulfur-based malodors

The above analytical test is repeated using samples containing an acid catalyst to test their effect on sulfur-based malodors. Specifically, a 1 µL aliquot of each of the following controls and acid catalyst samples are pipeted into individual 10 mL silanized headspace vials in duplicate: TC as a control; a 50/50 mixture of TC and each of the following acid catalysts at 0.04%, 0.10%, 0.43% in DPM, 1.02% in DPM, and 2.04% in DPM: phenol, mesitylenic acid, caprylic acid, succinic acid, pivalic acid, tiglic acid, and benzoic acid.

Fig. 1 demonstrates that low vapor pressure acid catalysts provide up to 3 times better reduction of sulfur-based malodors in comparison to the control.

### Analytical Test - Effect of aldehydes and acid catalyst on amine-based and sulfur-based malodors

The above analytical test is repeated using sample formulations containing aldehydes from Accords A, B, and C, in accordance with the present invention, and an acid catalyst, as outlined in Tables 10 and 11.

Tables 10 and 11 show that a perfume mixture having as little as 1% aldehyde along with 1.5% acid catalyst performs better at reducing butylamine and butanethiol than the same perfume mixture having 5% aldehyde.

**Table 10**

| **Formulation** | **% butylamine reduction at 40 secs.** | | **% butanethiol reduction at 30 mins.** | |
|---|---|---|---|---|
| Perfume Mixture w/ 5 % RA (Control) | 34 | - | 2 | - |
| Perfume Mixture w/1% RA and w/1.5% Benzoic Acid | 42 | +7 | 12 | +10 |
| Perfume Mixture w/ 3 % RA and w/ 1.5% Benzoic Acid | 36 | +2 | 14 | +11 |
| Perfume A Mixture w/ 5 % RA and w/ 1.5% Benzoic Acid | 41 | +7 | 10 | +5 |

**Table 11**

| **Formulation** | **% butylamine Reduction at 40 secs.** | | **% butanethiol reduction at 30 mins.** | |
|---|---|---|---|---|
| Perfume mixture w/ 5 % RA (Control) | 4.94 | - | 10.52 | - |
| Perfume mixture w/ 1% RA and w/ 1.5% Benzoic Acid | 11.61 | +6.67 | 18.82 | +8.30 |
| Perfume mixture w/ 3% RA and w/ 1.5% Benzoic Acid | 26.89 | +21.95 | 14.85 | +4.33 |
| Perfume mixture w/ 5% RA and w/ 1.5% Benzoic Acid | 20.27 | +15.33 | 16.84 | +6.32 |

### Sensory test - Effect on Trash Sulfur and Amine-based malodors, and in-use scent level

Composition A (Table 1) and Composition B (Table 2) are evaluated against sulfur and amine based trash odors according to the method described hereby:

### Fish Mix Preparation (Amine Based Malodor)

Use StarKist™ Solid White Albacore tuna in water, 142 g (5 oz) can

### Sample Prep: 13 Gal Trash Bag

1) Open tuna can and drain off water into sink (save can and the lid).
2) Empty tuna into a container (you will only need to keep 5 grams of the tuna; keep can).
3) Put the tuna can & lid in your sample trash bag and add 5 grams of tuna to the bag.
4) Put open sample bags in fume hood to age for 24 hours &/or 48 hours.
**Note:** Prep 48hr samples two days prior to efficacy test.

### Chicken Mix Preparation (Sulfur Based Malodors)

Use Tyson™ Boneless Breast only. Select packages containing lots of juice; usually ∼ 454 g (11b) w/3 breasts.

### Sample Prep: 13 Gal Trash Bag

1) Remove the diaper from underneath the chicken and weigh: Target is 100grams.
   A. Adjust diaper weight to target 100 grams.
      Add juice from the package to diaper if under 100grams.
      Squeeze out excess juice from diaper if over 100 grams.
   B. Put diaper into sample trash bag when weight is correct; 100gms.
2) Cut 25 grams of chicken up into small chunks and put into bag with diaper.
3) Put open sample bags in fume hood to age for 24 hours &/or 48 hours.
**Note:** Prep 48hr samples two days prior to efficacy test.

### Sample Evaluation Procedure: Post Aging 48hrs

1) Put open sample bags in tall kitchen trash can with top of bag stretched over top of can.
   A) Ensure bag is not tangled inside can so that trash mix is fully exposed.
   B) Cover top of open trash can with aluminum foil and seal around edges.
   C) Place prepped trash cans in vented area for evaluation.
   D) After a minimum of 15 minutes, instruct evaluator to evaluate trash odors by lifting one corner of the aluminum and sniffing.

### Note:

1) A reference control should be prepped and evaluated at same time as other samples. The control should be identified to the evaluator. Additional control reps can be included in the testing without letting the evaluator knowing which one(s) they are.
2) The intensity ratings are based on odor grading using the scale shown in Table 12.

**Table 12**

| **Expert Sensory Grader Odor Evaluation Scale** | |
|---|---|
| ***Score*** | ***Description corresponding to Score*** |
| 0 | No odor present |
| 10 | Very slight odor - "I think there is an odor present" |
| 20 | Slight odor - "I detect something but cannot identify specific odor" |
| 25 | Slight odor |
| 50 | Moderate |
| 75 | Strong odor |
| 100 | Extremely Strong odor |

Table 13 demonstrates that the unscented or low scented test mixture according to the present invention provides greater malodor reduction compared to an unscented Control but, notably, has equal or less perfume scent intensity.

**Table 13**

| | **Malodor Intensity** | | **Scent Intensity** | |
|---|---|---|---|---|
| | **Fish** | **Chicken** | **Fish** | **Chicken** |
| Control (unscented) | 53 | 71 | 28 | 28 |
| Composition A | 49 | 56 | 15 | 0 |
| Composition B | 37 | 55 | 8 | 35 |

### Sensory test - Scent Intensity and Malodor Efficacy of Low Scented Air Freshener

Malodor reduction compositions according the present invention are evaluated in a non-energized membrane type air freshener to determine their scent intensity and malodor efficacy.

To determine scent intensity, 5g of each test composition is placed into an empty Febreze® Set & Refresh™ non-energized air freshener utilizing a microporous membrane (Teslin 1100HD, PPG Industries Monroville, PA) with a surface area of approximately 34 cm². Samples are tested 24 hours after preparation, to ensure that the microporous membrane is fully saturated.

Evaluations are conducted in rooms with a floor size of 9.3 sq m (100 sq ft) and an 2.44 m (8 ft) ceiling height. Evaluation rooms are constructed with glass walls with aluminum framing. The rear wall and ceiling are constructed of aluminum composite panel to provide non-absorbent cleaning surfaces. The room temperature range is controlled to 21.1°C ± 1.7°C (70°F ± 3°F). The room relative humidity is controlled to 40%RH ± 5%. Air is supplied from the ceiling near the door and exhausted high and low from the rear wall. An air change rate of 2 air changes per hour is maintained during the test. A ceiling fan located in the center of the room is set to medium speed.

Samples are placed in the evaluation room in a visually shielded sample stand 1 hour before panelist evaluation. This time allows sample to diffuse effectively into the room.

Immediately after the 1hr dwell time is complete, panelists evaluate rooms in groups of 2-5 depending on the total number of panelists available. There are 4 groups of panelists per test. Panelists enter the rooms to make the evaluation. Panelists are instructed to walk around room and base their score on the average of the entire room. Panelists enter and exit the room together to minimize door seconds between samples.

Each panelist receives a ballot with the specified test codes and evaluation order. Panelists evaluate in a randomized order. Panelists mark their ballots by evaluating the room scent intensity on a 0-100 scale according to Table 12. The scores are tabulated and the average malodor intensity and scent intensity scores for each time interval are recorded.

Table 14 summarizes results of these intensity evaluations for compositions of the current invention and prior art. Compositions of the current invention show lower scent intensity compared to prior-art perfume compositions.

**Table 14**

| **Samples** | **In-room intensity @ 1 hr** |
|---|---|
| Blank (no sample) | 25 |
| | |
| Perfume * | 67 |
| 100% Malodor Reduction Composition A (from Table 1) | 59 |
| 100% Diluent (Dioctyl Adipate) | 30 |
| 90% Diluent (Dioctyl Adipate) | 27 |
| 10% Malodor Reduction Composition A (from Table 1) | |
| 10% Malodor Reduction Composition A (from Table 1) | 17 |
| 10% Perfume* | |
| 80% Diluent A | |
| 10% Malodor Reduction Composition A (from Table 1) | 24 |
| 20% Perfume* | |
| 70% Diluent A | |
| 20% Malodor Reduction Composition A (from Table 1) | 44 |
| 10% Perfume 2 | |
| 70% Diluent A | |
| 20% Malodor Reduction Composition A (from Table 1) | 35 |
| 20% Perfume* | |
| 60% Diluent A | |

| | |
|---|---|
| *Any desired perfume | |

To determine malodor reduction efficacy, malodor is prepared according to the following procedure:
Place Presto™ skillet into fume hood and turn on to 121.1°C (250°F). Place 80 grams of Crisco® oil into skillet and cover with skillet lid. Allow 10 minutes for equilibration. Remove skillet lid and check oil temperature with thermometer. Place 50 grams of chopped, commercially prepared garlic in water into skillet. Cover skillet with lid. Cook for 2.5 minutes or until garlic is translucent, with a portion staring to turn brown but not burn. Remove garlic from the skillet. Place 5 grams of garlic in each of 3 Petri dishes. Place covers on each Petri dish.

Malodor reduction efficacy is tested in a test chamber. Each test chamber is 99.70 cm (39.25 inches) wide, by 63.50 cm (25 inches) deep, by 54.61 cm (21.5 inches) high with a volume of 0.34 cubic meters (12.2 cubic feet). The test chamber can be purchased from Electro-Tech Systems, Glenside, PA. Each test chamber is equipped with a fan (Newark catalog #70K9932, 115 VAC, 90CFM) purchased from Newark Electronics, Chicago, IL.

Prepare Febreze® Set & Refresh™ non-energized air fresheners as described above, with 5g of the sample composition to be tested. Five minutes before cooked garlic is introduced to the test chambers, each non-energized air freshener is placed into individual test chambers on the opposite side of a small fan.

Place each covered Petri dish, with 5 grams of garlic, into an individual test chamber in front of the fan. Note: One test chamber will not contain a passive dispenser device. Remove the lids of the Petri dishes to expose contents for a dwell time sufficient to provide an initial odor intensity grade of 70-80 (about 2 minutes). Once the initial odor intensity grade has been reached in a test chamber, remove the Petri dish from the test chamber.

At pre-determined time intervals, trained evaluators open each chamber, smell the chamber for odor intensity, and assign a score for malodor intensity, based on the scale in Table 12. Immediately following, the trained evaluator smells the same chamber for perfume scent intensity, and assigns a score for scent intensity, *also* based on the scale in Table 12. The chamber door is closed between sequential evaluators. The scores are tabulated and the average malodor intensity and scent intensity scores for each time interval are recorded.

The time required for malodor intensity to be reduced below 20 according to the scale in Table 12 is considered the malodor removal time. Malodor reduction compositions of the current invention have malodor removal times comparable to a perfume composition of the prior art.

**Table 15**

| **Samples** | **Garlic Malodor Removal Time to malodor intensity <20.** |
|---|---|
| Perfume* | 28 minutes |
| 100% Malodor Reduction Composition A (from Table 1) | 26 minutes |
| 90% Diluent (Dioctyl Adipate) | 31 minutes |
| 10% Malodor Reduction Composition A (from Table 1) | |
| 90% Diluent (Iso-Nonyl Acetate) | 41 minutes |
| 10% Malodor Reduction Composition A (from Table 1) | |

| | |
|---|---|
| *Any desired perfume | |

While particular embodiments of the present invention have been illustrated and described, it would be obvious to those skilled in the art that various other changes and modifications can be made without departing from the scope of the invention. It is, therefore, intended to cover in the appended claims all such changes and modifications that are within the scope of this invention.

## Claims

1. A malodor reduction composition comprising:
a perfume mixture comprising an effective amount, preferably from 5% to 100% by weight of said malodor reduction composition, of methyl palmitate, farnesol, vetivert acetate, undecylenic aldehyde, terpinyl acetate, methyl Iso-eugenol, phenyl acetaldehyde dimethyl acetal, patchone and optionally a material selected from the group consisting of benzophenones, diphenyl Oxide, melozone, iso nonyl acetate, cedryl methyl ether and mixtures thereof, said malodor reduction composition or said perfume mixture being optionally encapsulated.

2. The malodor reduction composition of Claim 1 said composition comprising cedryl methyl ether, florhydral, helional, vertofix coeur, and mixtures thereof.

3. The malodor reduction composition of Claim 1 wherein said perfume mixture comprises at least one aldehyde selected from the group consisting of floral super, 2-ethoxy Benzylaldehyde, 2-isopropyl-5-methyl-2-hexenal, 5-methyl Furfural, 5-methyl-thiophene-carboxaldehyde, p-anisaldehyde, Benzylaldehyde, Cinnamic aldehyde, Decyl aldehyde, Ligustral, Lyral, Melonal, o-anisaldehyde, P.T. Bucinal, Thiophene carboxaldehyde, trans-4-Decenal, trans trans 2,4-Nonadienal, Undecyl aldehyde, and mixtures thereof

4. The malodor reduction composition of Claim 1 wherein said perfume mixture comprises 5% to 100% benzophenone, methyl palmitate, farnesol, vetivert acetate, and undecylenic aldehyde.

5. The malodor reduction composition of Claim 1 wherein said perfume mixture comprises an aldehyde mixture selected from the group consisting of Accord A, Accord B, Accord C, and mixtures thereof, preferably said perfume mixture comprises 1% to 10% of Accord A, by weight of said perfume mixture.

6. The malodor reduction composition of Claim 2 said composition comprising an acid catalyst present in an amount of 0.1% to 1.5%, by weight of said malodor reduction composition.

7. The malodor reduction composition of Claim 6 wherein said acid catalyst has a vapor pressure from 0.00133 to 26.7 mbar (0.001 to 20 torr) at 25°C, preferably said acid catalyst is a carboxylic acid, preferably said acid cataylst is 5-methyl thiophene carboxylic acid.

8. The malodor reduction composition of Claim 6 wherein said acid has a pKa of 1 to 7, from 3 to 6, or from 4 to 6.5.

9. The malodor reduction composition of Claim 1 said composition comprising an ingredient selected from the group consisting of: odor masking agents, odor blocking agents, diluents, and mixtures thereof.

10. A plastic film comprising the composition of Claim 1.

11. The plastic film of Claim 10 wherein said plastic film comprises LLDPE, LDPE, HDPE, and/or compostable film.

12. The plastic film of Claim 10 wherein said plastic film comprises 0.5 mg to 100 mg, preferably 5 mg to 30 mg, preferably 5 mg to 15 mg of said malodor reduction composition per 20 grams of said plastic film.

13. A method of controlling malodors comprising:
contacting the material comprising a malodor with the composition of Claim 1..

14. The method of Claim 13 wherein said contacting step comprises contacting said material containing a malodor with 1 mg to 50 mg, from 3 mg to 30 mg, or from 5 mg to 20 mg of said composition per 20 grams of said material containing a malodor.

## Patentansprüche

1. Zusammensetzung zur Verminderung übler Gerüche, umfassend:
ein Duftstoffgemisch, das eine wirksame Menge, vorzugsweise zwischen 5 Gew.-% und 100 Gew.-% der Zusammensetzung zur Verminderung übler Gerüche, aus Methylpalmitat, Farnesol, Vetiveracetat, Undecylenaldehyd, Terpinylacetat, Methyl-iso-eugenol, Phenyl-acetaldehyddimethylacetal, 4-tert-Butylcyclohexanol und optional einem Material ausgewählt aus der Gruppe, bestehend aus Benzophenonen, Diphenyloxid, Melozon, iso-Nonylacetat, Cedryl-methylether und Mischungen davon, umfasst, wobei die Zusammensetzung zur Verminderung übler Gerüche bzw. das Duftstoffgemisch wahlweise verkapselt sind.

2. Zusammensetzung zur Verminderung übler Gerüche nach Anspruch 1, wobei die Zusammensetzung Cedryl-methylether, Florhydral, Helional, Vertofix coeur und Mischungen davon umfasst.

3. Zusammensetzung zur Verminderung übler Gerüche nach Anspruch 1, wobei das Duftstoffgemisch wenigstens ein Aldehyd umfasst, ausgewählt aus der Gruppe, bestehend aus Floral Super, 2-Ethoxy-benzylaldehyd, 2-Isopropyl-5-methyl-2-hexenal, 5-Methylfurfural, 5-Methyl-thiophen-carboxaldehyd, p-Anisaldehyd, Benzylaldehyd, Zimtaldehyd, Decylaldehyd, Ligustral, Lyral, Melonal, o-Anisaldehyd, P.T. Bucinal, Thiophene-carboxaldehyd, trans-4-Decenal, trans-trans-2,4-Nonadienal, Undecylaldehyd und Mischungen davon.

4. Zusammensetzung zur Verminderung übler Gerüche nach Anspruch 1, wobei das Duftstoffgemisch 5 % bis 100 % Benzophenon, Methylpalmitat, Farnesol, Vetiveracetat und Undecylenaldehyd umfasst.

5. Zusammensetzung zur Verminderung übler Gerüche nach Anspruch 1, wobei das Duftstoffgemisch ein Aldehydgemisch umfasst, ausgewählt aus der Gruppe, bestehend aus Akkord A, Akkord B, Akkord C und Mischungen davon, vorzugsweise wobei das Duftstoffgemisch 1 Gew.-% bis 10 Gew.-% Akkord A, bezogen auf das Gewicht des Duftstoffgemischs, umfasst.

6. Zusammensetzung zur Verminderung übler Gerüche nach Anspruch 2, wobei die Zusammensetzung einen Säurekatalysator in einer Menge von 0,1 Gew.-% bis 1,5 Gew.-%, bezogen auf das Gewicht der Zusammensetzung zur Verminderung übler Gerüche, umfasst.

7. Zusammensetzung zur Verminderung übler Gerüche nach Anspruch 6, wobei der Säurekatalysator einen Dampfdruck von 0,00133 bis 26,7 hPa (von 0,00133 bis 26,7 mbar (0,001 bis 20 Torr)) bei 25 °C aufweist, vorzugsweise wobei es sich bei dem Säurekatalysator um eine Carbonsäure handelt, vorzugsweise wobei es sich bei dem Säurekatalysator um 5-Methyl-thiophencarbonsäure handelt.

8. Zusammensetzung zur Verminderung übler Gerüche nach Anspruch 6, wobei die Säure einen pKs-Wert von 1 bis 7, von 3 bis 6 oder von 4 bis 6,5 aufweist.

9. Zusammensetzung zur Verminderung übler Gerüche nach Anspruch 1, wobei die Zusammensetzung einen Bestandteil umfasst, ausgewählt aus der Gruppe bestehend aus: Geruchsmaskierungsmitteln, Geruchsblockiermitteln, Verdünnungsmitteln und Mischungen davon.

10. Kunststofffolie, die die Zusammensetzung von Anspruch 1 umfasst.

11. Kunststofffolie nach Anspruch 10, wobei die Kunststofffolie LLDPE, LDPE, HDPE und/oder kompostierbare Folie umfasst.

12. Kunststofffolie nach Anspruch 10, wobei die Kunststofffolie 0,5 mg bis 100 mg, vorzugsweise 5 mg bis 30 mg, vorzugsweise 5 mg bis 15 mg der Zusammensetzung zur Verminderung übler Gerüche pro 20 Gramm der Kunststofffolie umfasst.

13. Verfahren zur Geruchsbekämpfung, umfassend:
Inkontaktbringen des einen üblen Geruch enthaltenden Materials mit der Zusammensetzung von Anspruch 1.

14. Verfahren nach Anspruch 13, wobei der Schritt des Inkontaktbringens das Inkontaktbringen des einen üblen Geruch enthaltenden Materials mit 1 mg bis 50 mg, 3 mg bis 30 mg oder 5 mg bis 20 mg der Zusammensetzung pro 20 Gramm des einen üblen Geruch enthaltenden Materials umfasst.

## Revendications

1. Composition de réduction des mauvaises odeurs, comprenant :
un mélange parfumé comprenant une quantité efficace, de préférence de 5 % à 100 % en poids de ladite composition de réduction des mauvaises odeurs, de palmitate de méthyle, de farnésol, d'acétate de vétivert acétate de vétiver, d'aldéhyde undécylénique, d'acétate de terpinyle, de méthyliso-eugénol, d'acétate de terpinyle, de phényl-acétaldéhyde-diméthyl-acétal, de patchone et éventuellement d'un matériau choisi dans le groupe constitué de benzophénones, oxyde de diphényle, mélozone, acétate d'iso-nonyle, éther méthylique de cédryle et leurs mélanges, ladite composition de réduction des mauvaises odeurs ou ledit mélange parfumé étant éventuellement encapsulé.

2. Composition de réduction des mauvaises odeurs selon la revendication 1, ladite composition comprenant de l'éther méthylique de cédryle, du florhydral, de l'hélional, du vertofix coeur, et leurs mélanges.

3. Composition de réduction des mauvaises odeurs selon la revendication 1, dans laquelle ledit mélange parfumé comprend au moins un aldéhyde choisi dans le groupe constitué de floral super, 2-éthoxy benzylaldéhyde, 2-isopropyl-5-méthyl-2-hexénal, 5-méthyl furfural, 5-méthyl-thiophène-carboxaldéhyde, p-anisaldéhyde, benzylaldéhyde, aldéhyde cinnamique, décylaldéhyde, ligustral, lyral, mélonal, o-anisaldéhyde, P.T. bucinal, thiophène carboxaldéhyde, trans-4-décénal, trans trans 2,4-nonadiénal, aldéhyde undécylique, et leurs mélanges

4. Composition de réduction des mauvaises odeurs selon la revendication 1, dans laquelle ledit mélange parfumé comprend 5 % à 100 % de benzophénone, de palmitate de méthyle, de farnésol, d'acétate de vétiver et d'aldéhyde undécylénique.

5. Composition de réduction des mauvaises odeurs selon la revendication 1, dans laquelle ledit mélange parfumé comprend un mélange d'aldéhydes choisi dans le groupe constitué d'Accord A, Accord B, Accord C, et leurs mélanges, de préférence ledit mélange parfumé comprend 1 % à 10 % d'Accord A, en poids dudit mélange parfumé.

6. Composition de réduction des mauvaises odeurs selon la revendication 2, ladite composition comprenant un catalyseur acide présent en une quantité de 0,1 % à 1,5 % en poids de ladite composition de réduction des mauvaises odeurs.

7. Composition de réduction des mauvaises odeurs selon la revendication 6, dans laquelle ledit catalyseur acide a une pression de vapeur allant de 0,00133 à 26,7 hPa (de 0,00133 à 26,7 mbar (0,001 à 20 torr)) à 25 °C, de préférence ledit catalyseur acide est un acide carboxylique, de préférence ledit catalyseur acide est un acide 5-méthyl-thiophène carboxylique.

8. Composition de réduction des mauvaises odeurs selon la revendication 6, dans laquelle ledit acide a un pKa de 1 à 7, de 3 à 6, ou de 4 à 6,5.

9. Composition de réduction des mauvaises odeurs selon la revendication 1, ladite composition comprenant un ingrédient choisi dans le groupe constitué de : agents de masquage des odeurs, agents de blocage des odeurs, diluants, et leurs mélanges.

10. Film plastique comprenant la composition selon la revendication 1.

11. Film plastique selon la revendication 10, dans lequel ledit film plastique comprend du polyéthylène linéaire à basse densité, du polyéthylène à basse densité, du polyéthylène haute densité, et/ou un film compostable.

12. Film plastique selon la revendication 10, dans lequel ledit film plastique comprend 0,5 mg à 100 mg, de préférence 5 mg à 30 mg, de préférence 5 mg à 15 mg de ladite composition de réduction des mauvaises odeurs pour 20 grammes dudit film plastique.

13. Procédé de lutte contre les mauvaises odeurs, comprenant :
la mise en contact du matériau comprenant une mauvaise odeur avec la composition selon la revendication 1.

14. Procédé selon la revendication 13, dans lequel ladite étape de mise en contact comprend la mise en contact dudit matériau contenant une mauvaise odeur avec 1 mg à 50 mg, de 3 mg à 30 mg, ou de 5 mg à 20 mg de ladite composition pour 20 grammes dudit matériau contenant une mauvaise odeur.
